# EUROPEAN PATENT APPLICATION

(11) **EP 1 769 794 A1**
(43) Date of publication of application: **04.04.2007**
(21) Application number: 05766306.4
(22) Date of filing: 15.07.2005
(51) Int. Cl.: A61K 31/335, A61K 31/00, A61P 25/20, C07D 313/12

(54) **MEDICAMENT FOR PREVENTING AND/OR TREATING SLEEP DISORDER**

(30) Priority: 16.07.2004 JP 2004209308
(71) Applicant: KYOWA HAKKO KOGYO CO., LTD., Chiyoda-ku, Tokyo 100-8185 (JP)
(72) Inventor: TANAKA, Hisao, Niigata 940-0034 (JP); TANIGUCHI, Koji c/o.,Kyowa Hakko Kogyo LTD., Tokyo 100-8185 (JP)
(74) Representative: Casalonga, Axel
(86) International application number: PCT/JP2005/013114
(87) International publication number: WO 2006/009093

(57) **Abstract**

The present invention provides a preventive and/or therapeutic agent for sleeping disorder, which comprises, as an active ingredient, a dibenzo[b,e]oxepine derivative represented by formula (I): 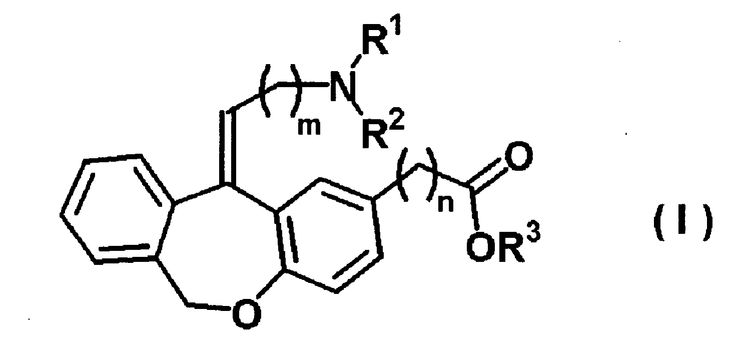 (wherein R¹, R², and R³ may be the same or different and each represents a hydrogen atom or lower alkyl, and m and n may be the same or different and each represents an integer of 1 to 4) or a pharmaceutically acceptable salt thereof.

## Description

### Technical Field

The present invention relates to a preventive and/or therapeutic agent for sleeping disorder and the like.

### Background Art

Sleeping disorder is defined as a general term of disorder of falling asleep, disorder of sleeping duration, disorder of hypersomnia, disorders that cause abnormal actions associated with sleeping and the like. For example, sleeplessness, sleep apnea syndrome and daytime hypersomnia caused by sleep apnea syndrome have been known (Merck Manual, 14th ed., chap. 173, 1999).

Sleeplessness is characterized by conditions such as incapability to fall asleep, difficulty in sleeping continually, incapability to obtain satisfactory sleep due to hindrance of sleeping pattern and the like, and in addition to mental stress, pains and the like, respiration disorder during sleep is said to be one of the causes for sleeplessness (Merck Manual, 14th ed., chap. 173, 1999).

The sleep apnea syndrome (SAS) has a basic feature that apnea (condition in which respiration stops for more than 10 seconds) or hypopnea (condition in which a ventilation amount is less than half of the ordinary respiration) occurs intermittently (Merck Manual 14th ed. chap. 173, 1999 and the like), and because of hypoxemia, hypercapnemia, respiratory acidosis, an awaking reaction and the like which are triggered by apnea or hypopnea, the disease induces various symptoms such as; complications of, for example, hypertension, cerebrovascular accidents and ischemic heart disease; mental disorders; or the like. The sleep apnea syndrome occurs most frequently among diseases showing daytime hypersomnia (sleepiness). A possibility of patients causing very serious accidents such as traffic accidents and accidents during labor due to sleepiness or a decrease in mental activities because of sleep apnea syndrome has been pointed out. Thus, interests to the sleep apnea syndrome have lately increased not only from the standpoint of the health of patients themselves but also of the social losses which may be caused by the patients ["Nippon Rinsho (Clinical Therapy)", 2000, vol. 58, p. 107].

The sleep apnea syndrome has now been defined generally as a syndrome having the symptoms that
(1) apneas are observed more than 20 to 30 times during one night's sleep (7 hours),
(2) a number of times apneas are observed per one hour sleep [an apnea index (AI)] is an average of 5 or more,
(3) a number of times apneas and hypopneas are observed per one hour sleep [an apnea-hypopnea index (AHI)] is an average of 10 or more, and the like.
   However, there are many cases that an arousals index per hour is high, though they do not satisfy the definitions mentioned above. Sleeping disorder due to obstruction of airway systems such as a nose is considered to be highly involved in such cases.

Examples of the sleep apnea syndrome include a syndrome caused by weakening of a respiratory center during sleep because of disorder in the respiratory center (central sleep apnea syndrome: CSAS), a syndrome that triggers airway obstruction by relaxation of an upper respiratory tract muscle during sleep (obstructive sleep apnea syndrome (OSAS)) and a mixed type of both cases (mixed sleep apnea syndrome: MSAS). Approximately half of patients with the sleep apnea syndrome are complicated by allergic rhinitis.

As a therapeutic method for sleep apnea syndrome, a nasal-continuous positive airway pressure (nasal-CPAP) method has probed to be effective, and it has been recognized as the first choice at present. As a therapeutic method after this method, installation of an oral appliance (OA), an upper respiratory tract surgical operation method and the like are mentioned. However, many patients feel pain in wearing a CPAP mask, and compliance in long-term use thereof is only about 70%. Regarding OA, an effective ratio is said to be about 60 to 70%, and there are many incomplete patients of the same because of pain in jaw or the like. Thus, the respective therapeutic methods have their own problems. Meanwhile, with respect to drug therapy, no high effectiveness has been currently established.

On the other hand, dibenzo[b,e]oxepine derivatives or pharmaceutically acceptable salts thereof are known as compounds having, for example, an antiallergic function, an antiinflammatory function, an antiasthmatic function, and the like (refer to Patent Documents 1, 2, 3, 4, 5, and 6, and Non-patent Document 1).

In particular, (Z)-11-(3-dimethylaminopropylidene)-6,11-dihydrodibenzo[b,e]oxepine-2-acetic acid (hereinafter referred to as "compound I") is known to have a histamine antagonism (refer to Non-patent Documents 2 and 3), an inhibitory action on chemical transmitter liberation (refer to Non-patent Document 4), an inhibitory action on inflammatory cytokine liberation (refer to Non-patent Document 5), an inhibitory action on tachykinin liberation (refer to Non-patent Document 6), and the like, and is clinically used as an antiallergic agent. It has also been found from a Phase III test that compound 1 has the function to highly improve the nasal congestion of allergic rhinitis (refer to Non-patent Document 7). It has been further known from a basic experiment using guinea pigs that compound 1 has an inhibitory action on a nasal congestion reaction in an experimental allergic rhinitis model (refer to Non-patent Document 8).
[Patent Document 1] Japanese Published Examined Patent Application No. 86925/1993
[Patent Document 2] US Patent No. 1282365
[Patent Document 3] Japanese Published Unexamined Patent Application No. 150082/1981
[Patent Document 4] Japanese Published,Unexamined Patent Application No. 126883/1983
[Patent Document 5] Japanese Published Unexamined Patent Application No. 227879/1984
[Patent Document 6] Japanese Published Unexamined Patent Application No. 28972/1985
[Non-patent Document 1] Journal of Medicinal Chemistry (J. Med. Chem.), 1978, vol. 21, pp. 633-639
[Non-patent Document 2] Clinical Pharmacology and Therapy (Yakuri to Rinsho), 1995, vol. 5, pp. 1817-1824
[Non-patent Document 3] Clinical Pharmacology and Therapy (Yakuri to Rinsho), 1995, vol. 5, pp. 1825-1835
[Non-patent Document 4] International Archives of Allergy and Immunology (Int. Arch. Allergy Immunol.), 1996, vol. 110, pp. 57-63
[Non-patent Document 5] Acta Ophthalmologica Scandinavia (Acta. Ophthalmol. Scand.), 1999, vol. 228, pp. 33-37
[Non-patent Document 6] Japanese Journal of Pharmacology (Jpn. J. Pharmacol.), 2001, vol. 117, pp. 967-973
[Non-patent Document 7] Otorhinolaryngology (Jibi), 1996, vol. 42, pp. 633-658
[Non-patent Document 8] Allergy in Practice (Allergy no Rinsho), 2003, vol. 23, pp. 63-75

### [Disclosure of Invention]

### [Problem to be Solved by the Invention]

An object of the present invention is to provide a preventive and/or therapeutic agent for sleeping disorder and the like, which comprises, as an active ingredient, a dibenzo[b,e]oxepine derivative or a pharmaceutically acceptable salt thereof.

### [Means for Solving the Problem]

The present invention relates to the following items (1) to (18):
(1) A preventive and/or therapeutic agent for sleeping disorder, which comprises, as an active ingredient, a dibenzo[b,e]oxepine derivative represented by formula (I) (hereinafter referred to as "compound (I)"): 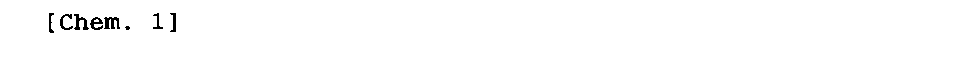 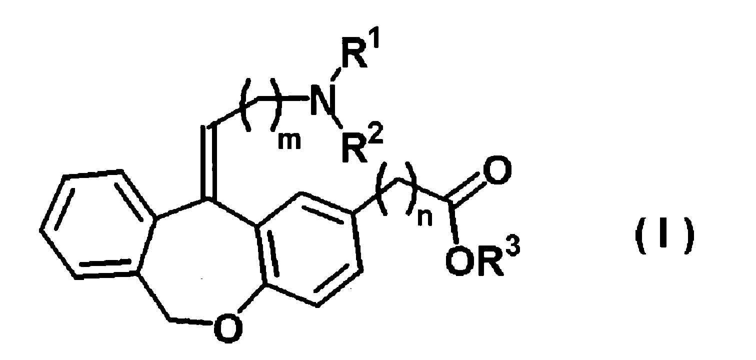 (wherein R¹, R², and R³ may be the same or different and each represents a hydrogen atom or lower alkyl, and m and n may be the same or different and each represents an integer of 1 to 4) or a pharmaceutically acceptable salt thereof.
(2) A preventive and/or therapeutic agent for sleeping disorder, which comprises, as an active ingredient, (Z)-11-(3-dimethylaminopropylidene)-6,11-dihydrodibenzo[b,e]oxepine-2-acetic acid represented by formula (IA): 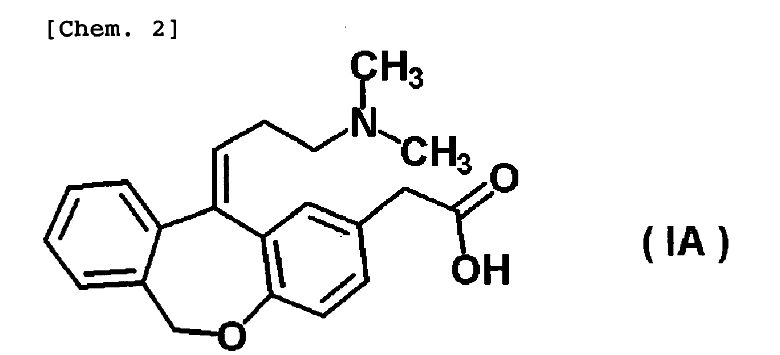 or a pharmaceutically acceptable salt thereof.
(3) The preventive and/or therapeutic agent for sleeping disorder according to (1) or (2), wherein the sleeping disorder is a disorder selected from the group consisting of disorder of falling asleep, disorder of sleeping duration and disorder of hypersomnia.
(4) The preventive and/or therapeutic agent for sleeping disorder according to (1) or (2), wherein the sleeping disorder is a sleep apnea syndrome.
(5) The preventive and/or therapeutic agent for sleeping disorder according to (4), wherein the sleep apnea syndrome is a sleep apnea syndrome complicated by allergic rhinitis.
(6) The preventive and/or therapeutic agent for sleeping disorder according to (4), wherein the sleep apnea syndrome is a sleep apnea syndrome which is not complicated by allergic rhinitis.
(7) A preventive and/or therapeutic method for sleeping disorder, which comprises administering an effective amount of a dibenzo[b,e]oxepine derivative represented by formula (I): 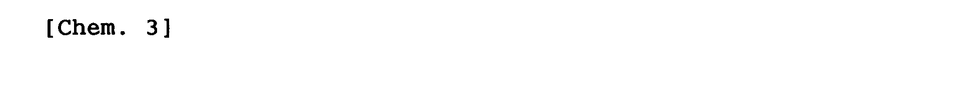 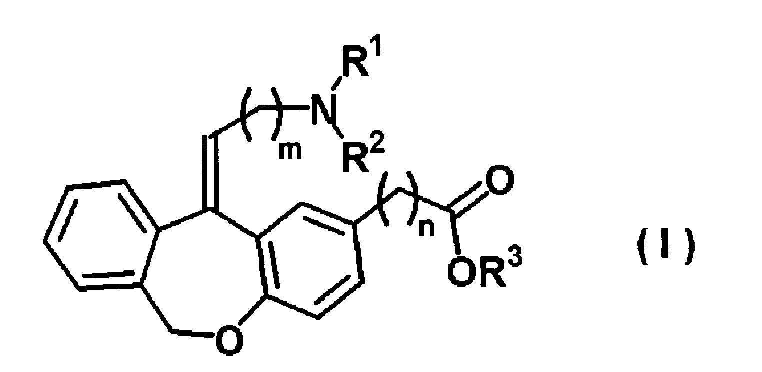 (wherein R¹, R² , R³, m and n have the same definitions as described above, respectively) or a pharmaceutically acceptable salt thereof.
(8) A preventive and/or therapeutic method for sleeping disorder, which comprises administering an effective amount of (Z)-11-(3-dimethylaminopropylidene)-6,11-dihydrodibenzo[b,e]oxepine-2-acetic acid represented by formula (IA): 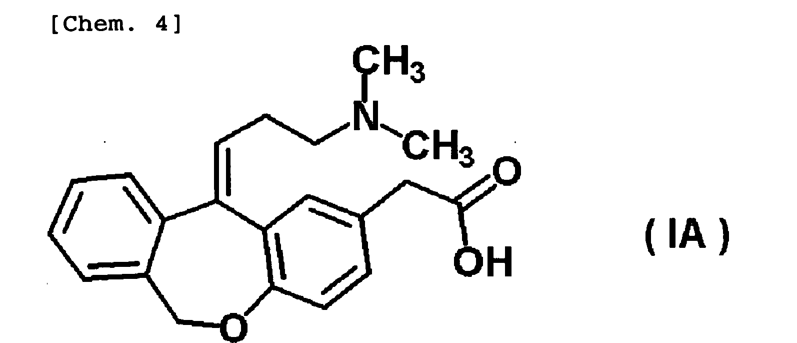 or a pharmaceutically acceptable salt thereof.
(9) The preventive and/or therapeutic method for sleeping disorder according to (7) or (8), wherein the sleeping disorder is a disorder selected from the group consisting of disorder of falling asleep, disorder of sleeping duration and disorder of hypersomnia.
(10) The preventive and/or therapeutic method for sleeping disorder according to (7) or (8), wherein the sleeping disorder is a sleep apnea syndrome.
(11) The preventive and/or therapeutic method for sleeping disorder according to (10), wherein the sleep apnea syndrome is a sleep apnea syndrome complicated by allergic rhinitis.
(12) The preventive and/or therapeutic method for sleeping disorder according to (10), wherein the sleep apnea syndrome is a sleep apnea syndrome which is not complicated by allergic rhinitis.
(13) Use of a dibenzo[b,e]oxepine derivative represented by formula (I): 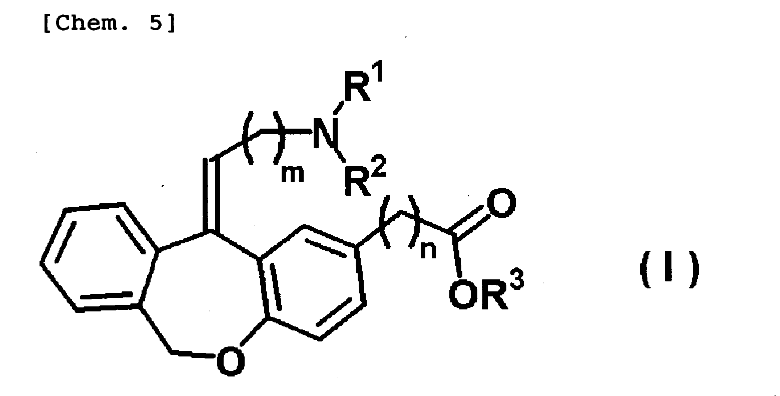 (wherein R¹, R², R³, m and n have the same definitions as described above, respectively) or a pharmaceutically acceptable salt thereof for the,manufacture of a preventive and/or therapeutic agent for sleeping disorder.
(14) Use of (Z)-11-(3-dimethylaminopropylidene)-6,11-dihydrodibenzo [b,e]oxepine-2-acetic acid represented by formula (IA): 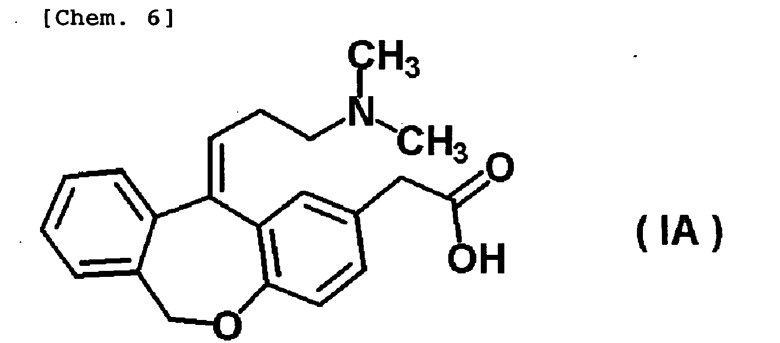 or a pharmaceutically acceptable salt thereof, for the manufacture of a preventive and/or therapeutic agent for sleeping disorder.
(15) The use according to (13) or (14), wherein the sleeping disorder is a disorder selected from the group consisting of disorder of falling asleep, disorder of sleeping duration and disorder of hypersomnia.
(16) The use according to (13) or (14), wherein the sleeping disorder is a sleep apnea syndrome.
(17) The use according to (16), wherein the sleep apnea syndrome is a sleep apnea syndrome complicated by allergic rhinitis.
(18) The use according to (16), wherein the sleep apnea syndrome is a sleep apnea syndrome which is not complicated by allergic rhinitis.

### [Effect of Invention]

The present invention provides a preventive and/or therapeutic agent for sleeping disorder and the like, which comprises, as an active ingredient, a dibenzo[b,e]oxepine derivative or a pharmaceutically acceptable salt thereof.

### [Best Mode for Carrying Out the Invention]

Examples of lower alkyl in the definition of each group in formula (I) include linear or branched alkyl having 1 to 8 carton atoms, such as methyl, ethyl, propyl, isopropyl butyl, isobutyl, sec-butyl, tert-butyl, pentyl, hexyl, heptyl, octyl, and the like.

Examples of pharmaceutically acceptable salts of compound (I) or compound 1 include pharmaceutically acceptable acid addition salts, metal salts, ammonium salts, organic amine addition salts, amino acid addition salts, and the like.

Examples of pharmaceutically acceptable acid addition salts of compound (I) or compound 1 include inorganic acid salts, such as hydrochlorides, sulfates, and phosphates; organic acid salts, such as acetates, maleates, fumarates, tartrates, citrates, and methanesulfonates. Examples of pharmaceutically acceptable metal salts include alkali metal salts, such as sodium salts, and potassium salts; alkaline-earth metal salts, such as magnesium salts, and calcium salts; aluminum salts; zinc salts and the like. Examples of pharmaceutically acceptable ammonium salts include ammonium salts, tetramethylammonium salts, and the like. Examples of pharmaceutically acceptable organic amine addition salts include addition salts of morpholine, piperidine, and the like. Examples of pharmaceutically acceptable amino acid addition salts include addition salts of lysine, glycine, phenylalanine, aspartic acid, glutamic acid, and the like. Among these salts, monohydrochlorides are more preferred.

Although a method for producing compound (I) and compound 1 is not particularly limited, compound (I) and compound 1 can be obtained by the method described in, for example, Japanese Published Examined Patent Application No. 86925/1993 or a similar method thereto. The resulting intermediate can be supplied to a subsequent reaction without particular purification.

The aimed compound of the production method can be isolated and purified by subjecting to a purification method generally used in synthetic organic chemistry, such as filtration, extraction, washing, drying, concentration, recrystallization, and various types of chromatography.

To obtain a salt of compound (I) or compound 1, when compound (I) or compound 1 is obtained in the form of a salt, the salt may be purified as it is. Further, when compound (I) or compound 1 is obtained in a free form, compound (I) or compound 1 may be dissolved or suspended in a suitable solvent, followed by addition of an acid or a base to form a salt.

Compound (I) or compound 1 and pharmaceutically acceptable salts thereof may exist in the form of an adduct with water or any of various solvents. The adduct can also be used for the preventive and/or therapeutic agent for sleeping disorder of the present invention.

Examples of the sleeping disorder to be prevented or treated in the present invention include sleeping disorder comprising one or more disorders selected from disorder of falling asleep, disorder of sleeping duration, disorder of hypersomnia, disorders that cause abnormal actions associated with sleeping and the like [for example, sleeplessness, sleep apnea syndrome and daytime hypersomnia caused by sleep apnea syndrome], symptoms accompanying these (for example, snore, obstruction of an airway system such as a nose, apnea, hypopnea, arousals and daytime sleepiness) and the like. Examples of the sleep apnea syndrome include central, obstructive and mixed sleep apnea syndromes and the like which are generally defined as the sleep apnea syndrome. Of these sleeping disorders, the obstructive or mixed sleep apnea syndrome or the disorder caused by airway obstruction, which includes the sleeping disorder caused by obstruction of the airway system such as a nose with high arousals index, is preferably prevented and/or treated. Further, among the sleep apnea syndrome, the sleep apnea syndrome complicated by allergic rhinitis and considered to result from nasal obstruction, the sleep apnea syndrome which is not complicated by allergic rhinitis, which is complicated by airway obstruction by relaxation of an upper respiratory tract muscle during sleep because of non-anallergic rhinitis, morphological abnormality such as tonsillar hypertrophy, obesity, polyp or hypertrophy of nose mucosa, and the like are preferably prevented and/or treated.

The preventive or therapeutic agent for sleeping disorder of the present invention can prevent, improve or treat symptoms associated with sleeping disorder (for example, symptoms which are secondarily triggered, such as daytime somnolence, decrease in intelligence, change in personality, reduction in concentration ability, morning headache, hallucination, automatism, dyspnea and cerebrovascular accidents).

Compound (I), compound 1, or a pharmaceutically acceptable salt thereof can be administered alone but is preferably provided as various general pharmaceutical preparations. The pharmaceutical preparations are used for animals and humans.

A pharmaceutical preparation according to the present invention may comprise, as an active ingredient, compound (I), compound 1 or pharmaceutically acceptable salts thereof alone or as a mixture with any other effective ingredients. In addition, such a pharmaceutical preparation is prepared by mixing the active ingredient with one or more pharmaceutically acceptable carriers and then subjecting the resulting mixture to any method well known in the technical field of pharmaceutics.

Examples of other effective ingredients to be incorporated in the pharmaceutical preparation according to the present invention include a nasal steroid and the like.

As for the administration route, it is preferred to select the most effective route of administration. Examples of the administration route include oral administration and parenteral administration such as hypodermic administration, intravenous administration, intranasal administration, and the like.

Examples of a dosage form include a tablet, a powder, granules, syrup, an injection, and the like.

A liquid preparation suitable for oral administration, for example, syrup, can be produced using water, a saccharide such as sucrose, sorbit, fructose, glycol such as polyethylene glycol or propylene glycol, oil such as sesame oil, olive oil, or soybean oil, a preservative such as p-hydroxybenzoate, and a flavor such as a strawberry flavor or peppermint and the like. A tablet, a powder, granules and the like can be produced by using an excipient such as lactose, glucose, sucrose, or mannitol, a disintegrator such as starch or sodium alginate, a lubricant such as magnesium stearate or talc, a binder such as polyvinyl alcohol, hydroxypropyl cellulose, or gelatin, a surfactant such as a fatty acid ester, a plasticizer such as glycerin, and the like.

An injection for hypodermic or intravenous administration (including continuous administration) is prepared as, for example, a solution, a suspension, an emulsion, or a solid injection used by dissolving or suspending in a solvent when used. A solution, a suspension, or an emulsion is prepared by dissolving, suspending, or emulsifying compound (I), compound 1, or pharmaceutically acceptable salts thereof in a solvent. Examples of the solvent include distilled water for injection, physiological saline, a buffer (such as phosphate buffer), vegetable oil, propylene glycol, polyethylene glycol, alcohols such as ethanol, and mixtures thereof. Such an injection may further contain a stabilizer, a solubilizing agent (such as glutamic acid, aspartic acid, Polysorbate 80 (trade mark), or the like), a suspending agent, an emulsifier, a soothing agent, a buffer, a preservative, or the like. In a final step, they are sterilized or subjected to aseptic operation to produce the injection. A sterile solid preparation, for example, a freeze-dried product and the like, may be produced so that it can be used by dissolving or suspending in sterile distilled water for injection or other solvent before administration.

An external preparation for intranasal administration is, for example, a liquid for external use and the like. Such an external preparation is prepared as a solution, a suspension, an emulsion, or a solid preparation used by dissolving or suspending in a solvent when used or the like. A solution, a suspension, or an emulsion can be prepared by dissolving, suspending, or emulsifying compound (I), compound 1 or pharmaceutically acceptable salts thereof in a solvent. Examples of the solvent include physiological saline, a buffer (such as phosphate buffer), propylene glycol, polyethylene glycol, and the like, or mixtures thereof. Such a liquid for external use may further contain a stabilizer, a solubilizing agent (such as glutamic acid or aspartic acid), a suspending agent (such as a polymer imparting viscosity, including Polysorbate 80 (trade mark), carboxymethyl cellulose, polyvinyl alcohol or the like), an emulsifier, a buffer, a preservative, or the like.

The doses and frequency of administration of compound (I), compound 1, or pharmaceutically acceptable salts thereof may vary depending on the dosage form, the age and body weight of a patient, the nature or seriousness of a symptom to be treated, and the like. In oral administration, in general, a dose of 0.01 mg to 1 g, preferably 0.05 to 50 mg, is administered to an adult once or several times a day. In parenteral administration such as intravenous administration, a dose of 0.001 mg to 100 mg, preferably 0.01 mg to 10 mg, is administered to an adult once or several times a day. However, these dosages and frequencies of administration vary with the various conditions described above.

Next, the effect of compound (I) on sleeping disorder will be described in detail below with reference to test examples.

### Test Example 1: Effect of olopatadine hydrochloride on sleeping disorder

Olopatadine hydrochloride was orally administered at a dose of 10 mg/day [a 5-mg tablet of Allelock (registered trademark: Kyowa Hakko Kogyo Co., Ltd.) was administered everyday, twice a day, with one tablet for each administration] to three patients (patient Nos. 1 to 3) with sleep apnea syndrome complicated by allergic rhinitis and seven patients (patient Nos. 4 to 10) with sleeping disorder which is not complicated by allergic rhinitis including four patients (patient Nos. 4 to 6 and 8) with sleep apnea syndrome which is not complicated by allergic rhinitis. Before and after the administration of olopatadine hydrochloride, an apnea-hypopnea index (AHI), an apnea index (AI) and an arousals index per hour were measured respectively by polysonography (PSG) for each patient. Further, before and after the administration of olopatadine hydrochloride, a test for daytime Epworth sleepness scale was performed according to the following.

The presence or absence of allergic rhinitis was confirmed by performing RAST (radioallergosorbent test) for 12 types of antigens [house dust, *Dermatophagoides pteronyssinus,* Japanese cedar, Hinoki cypress, Japanese alder, ragweed, mugwort, goldenrod, Cocksfoot *(Dactylis glomerata), Candida albicans,* mold and animal epithelium].

The patients uncomplicated by allergic rhinitis had airway obstruction caused by non-atopic rhinitis.

### <ESS test>

In the following conditions 1 to 8, sleepiness was determined according to the next four levels, and seriousness of daytime sleepiness was evaluated by the total (ESS) of the points. A case in which ESS is 10 points or less is evaluated as normal.

### [Evaluation points]

0 point = does not sleep
1 point = rarely sleeps
2 points = sometimes sleeps
3 points = often sleeps

### [Conditions]

1. when sitting and reading a book
2. when watching television
3. when sitting in a public place without movement (for example, in a theater or a conference room)
4. when being in a car as a fellow passenger for an hour without rest
5. when lying and taking rest in a free time during afternoon
6. when sitting and talking with somebody
7. when sitting quietly after lunch without alcohol
8. when staying in a car for a few minutes because of a traffic jam

The effect of the agent was estimated according to the following standard.

### Determination standard:

A case in which symptoms (for example, snore, apnea, daytime sleepiness and the like) of a chief complaint from a patient or his family in the first medical examination (namely before administration of olopatadine hydrochloride) were completely eliminated after administration of olopatadine hydrochloride was determined as "markedly improved", and a case in which the symptoms were reduced by half was determined as "improved". Incidentally, as objective assessment, improved conditions in values of AHI, AI, arousals index and ESS were used for reference.

Profiles of patients complicated by allergic rhinitis to whom olopatadine hydrochloride was administered and conditions for administration of olopatadine hydrochloride to the patients are shown in Table 1-1, and profiles of patients uncomplicated by allergic rhinitis to whom olopatadine hydrochloride was administered and conditions for administration of olopatadine hydrochloride to the patients are shown in Table 2-1. Further, the results of the PSG measurement and the ESS test are shown in Tables 1-2 and 2-2 respectively.

### [Table 1-1]

**Table 1-1:**

| Profiles of patients complicated by allergic rhinitis and conditions for administration of olopatadine hydrochloride | | | | | | |
|---|---|---|---|---|---|---|
| Patient No. | Age | Sex | RAST examination result* | Chief complaint | Administration schedule of olopatadine hydrochloride | Administration period (day) |
| 1 | 72 | female | + | Snore, apnea | Morning and before sleep | 342 |
| 2 | 55 | female | + | Snore, apnea | Morning and before sleep | 160 |
| 3 | 38 | male | + | Snore, apnea, daytime sleepiness | Evening and before sleep | 177 |

| | | | | | | |
|---|---|---|---|---|---|---|
| * +:positive | | | | | | |

**[Table 1-2]**

| Table 1-2: Effect of olopatadine hydrochloride on sleeping disorder | | | | | | | | | |
|---|---|---|---|---|---|---|---|---|---|
| Patient No. | AHI | | Al | | Arousals Index | | ESS | | Estimation of effect |
| | Before therapy | After therapy | Before therapy | After therapy | Before therapy | After therapy | Before therapy | After therapy | |
| 1 | 33.0 | 5.8 | 10.2 | 4.3 | 12.4 | 10.3 | 16 | 12 | Improved |
| 2 | 29.6 | 2.7 | 22.7 | 1.5 | 27.9 | 4.4 | 19 | 11 | Improved |
| 3 | 58.7 | 11.1 | 55.7 | 7.6 | 44.1 | 18.9 | 18 | 9 | Markedly improved |

From the results of Table 1-2, it has been found that olopatadine hydrochloride improves symptoms of sleeping disorder such as snore, apnea, hypopnea, arousals and daytime sleepiness in the patients with sleep apnea syndrome complicated by allergic rhinitis and has a clinical effect to sleep apnea syndrome complicated by allergic rhinitis.

### [Table 2-1]

**Table 2-1:**

| Profiles of patients uncomplicated by allergic rhinitis and conditions for administration of olopatadine hydrochloride | | | | | | |
|---|---|---|---|---|---|---|
| Patient No. | Age | Sex | RAST examination result* | Chief complaint | Administration schedule of olopatadine hydrochloride | Administration period (day) |
| 4 | 20 | female | - | Snore, daytime sleepiness, morning headache | Morning and evening | 74 |
| 5 | 56 | male | - | Snore, apnea | Morning and evening | 125 |
| 6 | 66 | male | - | Snore, apnea | Morning and evening | 124 |
| 7 | 56 | female | - | Snore, apnea, daytime sleepiness | Evening and before sleep | 106 |
| 8 | 63 | male | - | Snore, apnea | Evening and before sleep | 96 |
| 9 | 59 | male | - | Snore, daytime sleepiness | Evening and before sleep | 91 |
| 10 | 48 | female | - | Snore, daytime sleepiness | Evening and before sleep | 160 |

| | | | | | | |
|---|---|---|---|---|---|---|
| * -: negative | | | | | | |

**[Table 2-2]**

| Table 2-2: Effect of olopatadine hydrochloride on sleeping disorder. | | | | | | | | | |
|---|---|---|---|---|---|---|---|---|---|
| Patient No. | AHI | | Al | | Arousals Index | | ESS | | Estimation of effect |
| | Before therapy | After therapy | Before therapy | After therapy | Before therapy | After therapy | Before therapy | After therapy | |
| 4 | 12.8 | 1.8 | 11.8 | 0.8 | 23.9 | 2.1 1 | 18 | 9 | Markedly improved |
| 5 | 13.7 | 3.4 | 10.4 | 2.8 | 21.2 | 4.9 | 18 | 12 | Improved |
| 6 | 16.5 | 4.2 | 12.8 | 3.4 | 23.6 | 5.9 | 20 | 11 | Improved |
| 7 | 4.8 | 2.6 | 2.4 | 1.2 | 17.5 | 2.6 | 16 | 10 | Markedly improved |
| 8 | 21.3 | 4.5 | 16.5 | 3.1 | 28.9 | 5.3 | 18 | 10 | Improved |
| 9 | 3.8 | 1.3 | 3.2 | 0.8 | 15.6 | 2.3 | 17 | 8 | Markedly improved |
| 10 | 4.9 | 0.9 | 4.1 | 0.4 | 13.6 | 1.8 | 19 | 9 | Markedly improved |

From the results of Table 2-2, it has been found that olopatadine hydrochloride improves symptoms of sleeping disorder such as snore, apnea, hypopnea, arousals, daytime sleepiness and morning headache in patients with sleeping disorder which is not complicated by allergic rhinitis and has a clinical effect to sleeping disorder which is not complicated by allergic rhinitis.

From the above results, it has been found that olopatadine hydrochloride improves symptoms of sleeping disorder such as snore, apnea, hypopnea, arousals, daytime sleepiness and morning headache and has the clinical effect against sleeping disorder such as sleep apnea syndrome, regardless of the presence or absence of complication by allergic rhinitis.

Although the present invention will be described in detail below with reference to examples, the present invention is not limited to these examples.

### [Example 1]

Tablet: A tablet having the following composition is prepared by a conventional method.

| Prescription | |
|---|---|
| Monohydrochloride of compound | 1: 5 mg |
| Lactose: | 61 mg |
| Potato starch: | 30 mg |
| Polyvinyl alcohol: | 3 mg |
| Magnesium stearate: | 1 mg |
| | 100 mg |

### [Example 2]

Powder: A powder having the following composition is prepared by a conventional method.

| Prescription | |
|---|---|
| Compound 1: | 10 mg |
| Lactose: | 190 mg |
| | 200 mg |

### [Example 3]

Injection: An injection having the following composition is prepared by a conventional method.

| Prescription | |
|---|---|
| Compound 1: | 2 mg |
| Purified soybean oil: | 200 mg |
| Purified egg-yolk lecithin: | 24 mg |
| Glycerin for injection: | 50 mg |
| Distilled water for injection: | 1.72 mL |
| | 2.00 mL |

### [Example 4]

Syrup: Syrup having the following composition is prepared by a conventional method.

| Prescription | |
|---|---|
| Compound 1: | 300 mg |
| Purified sucrose: | 40 g |
| Methyl paraoxybenzoate: | 40 mg |
| Ethyl paraoxybenzoate: | 10 mg |
| Strawberry flavor: | 1 mL |
| Distilled water: | proper amount |
| | 100.00 mL |

### Industrial Applicability

The present invention provides a preventive and/or therapeutic agent for sleeping disorder and the like, which comprises, as an active ingredient, a dibenzo[b,e]oxepine derivative or a pharmaceutically acceptable salt thereof.

## Claims

1. A preventive and/or therapeutic agent for sleeping disorder, which comprises, as an active ingredient, a dibenzo[b,e]oxepine derivative represented by formula (I): 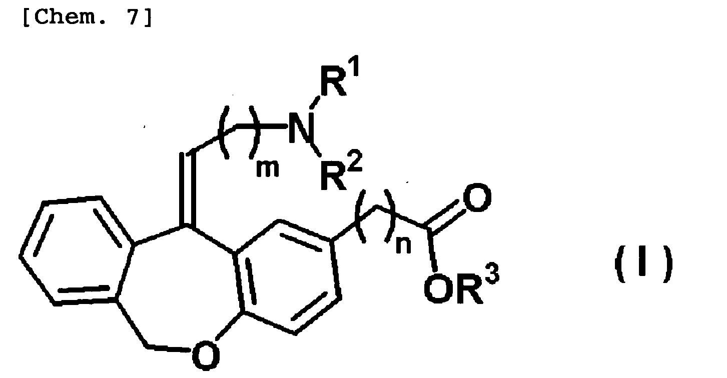 (wherein R¹, R², and R³ may be the same or different and each represents a hydrogen atom or lower alkyl, and m and n may be the same or different and each represents an integer of 1 to 4) or a pharmaceutically acceptable salt thereof.

2. A preventive and/or therapeutic agent for sleeping disorder, which comprises, as an active ingredient, (Z)-11-(3-dimethylaminopropylidene)-6,11-dihydrodibenzo[b,e]oxepine-2-acetic acid represented by formula (IA): 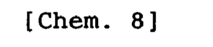 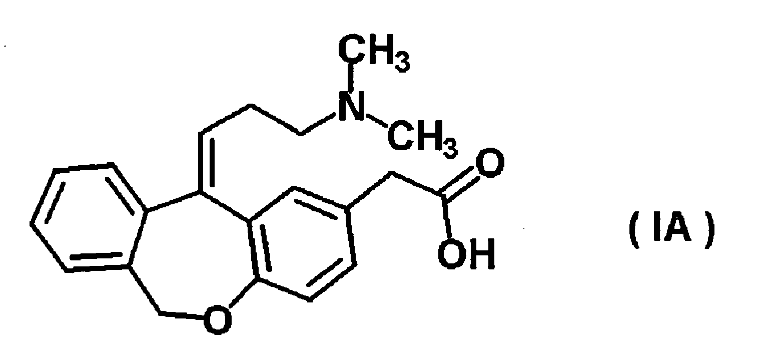 or a pharmaceutically acceptable salt thereof.

3. The preventive and/or therapeutic agent for sleeping disorder according to Claim 1 or 2, wherein the sleeping disorder is a disorder selected from the group consisting of disorder of falling asleep, disorder of sleeping duration and disorder of hypersomnia.

4. The preventive and/or therapeutic agent for sleeping disorder according to Claim 1 or 2, wherein the sleeping disorder is a sleep apnea syndrome.

5. The preventive and/or therapeutic agent for sleeping disorder according to Claim 4, wherein the sleep apnea syndrome is a sleep apnea syndrome complicated by allergic rhinitis.

6. The preventive and/or therapeutic agent for sleeping disorder according to Claim 4, wherein the sleep apnea syndrome is a sleep apnea syndrome which is not complicated by allergic rhinitis.

7. A preventive and/or therapeutic method for sleeping disorder, which comprises administering an effective amount of a dibenzo[b,e]oxepine derivative represented by formula (I): 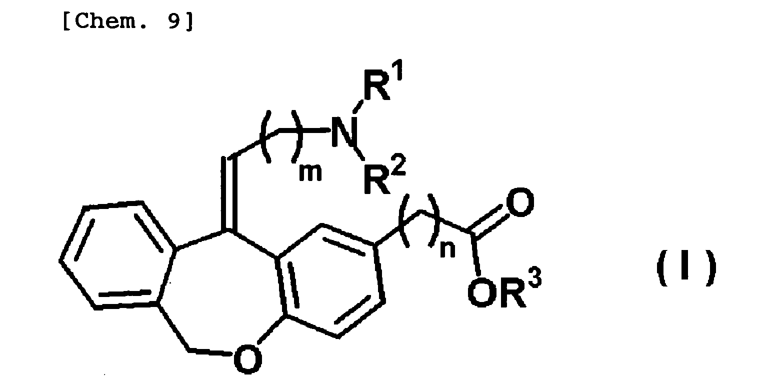 (wherein R¹, R², R³, m and n have the same definitions as described above, respectively) or a pharmaceutically acceptable salt thereof.

8. A preventive and/or therapeutic method for sleeping disorder, which comprises administering an effective amount of (Z)-11-(3-dimethylaminopropylidene)-6,11-dihydrodibenzo[b,e]oxepine-2-acetic acid represented by formula (IA): 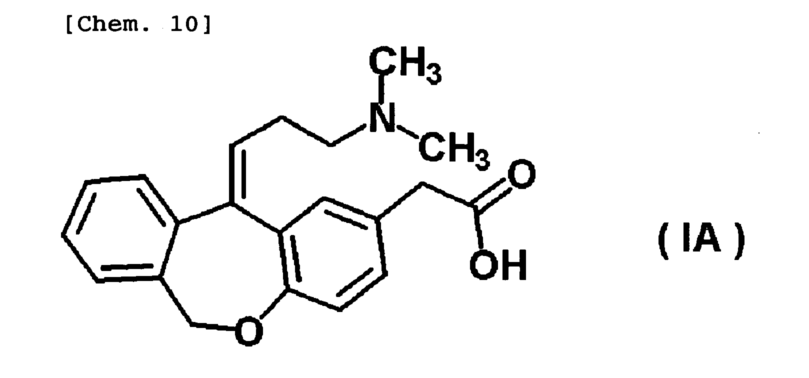 or a pharmaceutically acceptable salt thereof.

9. The preventive and/or therapeutic method for sleeping disorder according to Claim 7 or 8, wherein the sleeping disorder is a disorder selected from the group consisting of disorder of falling asleep, disorder of sleeping duration and disorder of hypersomnia.

10. The preventive and/or therapeutic method for sleeping disorder according to Claim 7 or 8, wherein the sleeping disorder is a sleep apnea syndrome.

11. The preventive and/or therapeutic method for sleeping disorder according to Claim 10, wherein the sleep apnea syndrome is a sleep apnea syndrome complicated by allergic rhinitis.

12. The preventive and/or therapeutic method for sleeping disorder according to Claim 10, wherein the sleep apnea syndrome is a sleep apnea syndrome which is not complicated by allergic rhinitis.

13. Use of a dibenzo[b,e]oxepine derivative represented by formula (I): 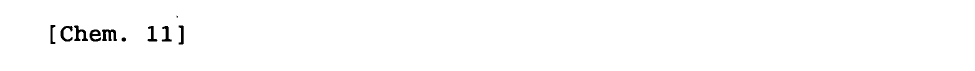 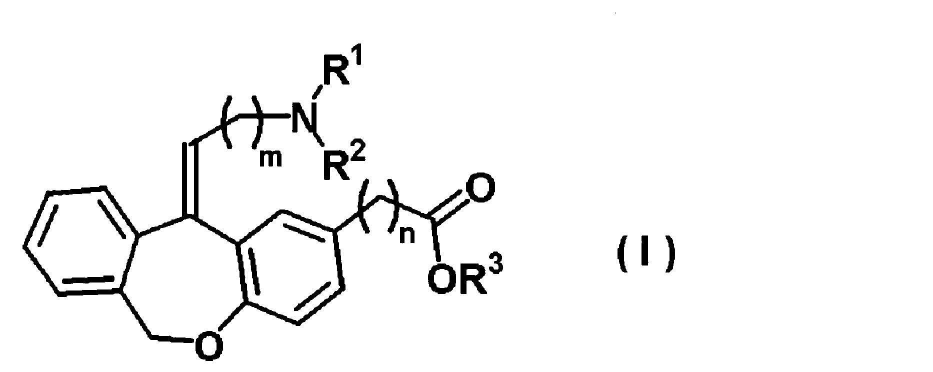 (wherein R¹, R², R³, m and n have the same definitions as described above, respectively) or a pharmaceutically acceptable salt thereof for the manufacture of a preventive and/or therapeutic agent for sleeping disorder.

14. Use of (Z)-11-(3-dimethylaminopropylidene)-6,11-dihydrodibenzo[b,e]oxepine-2-acetic acid represented by formula (IA): 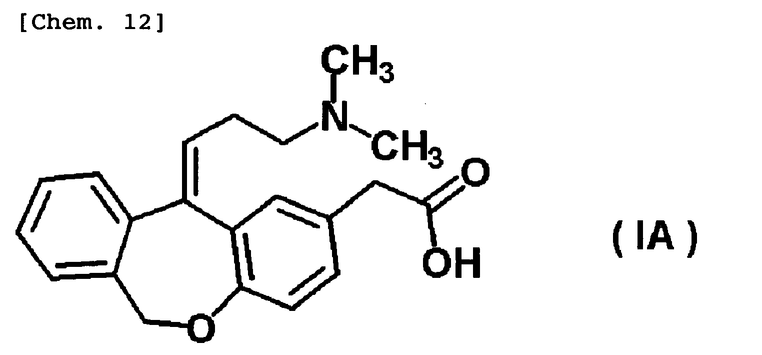 or a pharmaceutically acceptable salt thereof, for the manufacture of a preventive and/or therapeutic agent for sleeping disorder.

15. The use according to Claim 13 or 14, wherein the sleeping disorder is a disorder selected from the group consisting of disorder of falling asleep, disorder of sleeping duration and disorder of hypersomnia.

16. The use according to Claim 13 or 14, wherein the sleeping disorder is a sleep apnea syndrome.

17. The use according to Claim 16, wherein the sleep apnea syndrome is a sleep apnea syndrome complicated by allergic rhinitis.

18. The use according to Claim 16, wherein the sleep apnea syndrome is a sleep apnea syndrome which is not complicated by allergic rhinitis.
